# EUROPEAN PATENT APPLICATION

(11) **EP 4 763 995 A2**
(43) Date of publication of application: **24.06.2026**
(21) Application number: 26173578.1
(22) Date of filing: 17.05.2022
(51) Int. Cl.: C12Q 1/6806

(54) **PRE-TEMPLATED INSTANT PARTITIONS FOR SCREENING**

(30) Priority: 18.05.2021 US 202163190058 P
(62) Divisional of application: 22805318.7
(71) Applicant: Illumina, Inc., San Diego, CA 92122 (US)
(72) Inventor: FONTANEZ, Kristina, Massachusetts 02476 (US); D'AMATO, Christopher, Massachusetts 02481 (US); CLARK, Iain, Massachusetts 02472 (US)
(74) Representative: Graham Watt & Co

(57) **Abstract**

The invention provides high-throughput systems and methods for screening CRISPR- edited cells in bulk with single cell resolution. Methods of the invention use cells expressing polyadenylated guide RNAs that are detectable by RNA sequencing. Methods of the invention provide for the detection of each cell's guide RNA along with its single cell transcriptome to provide useful gene expression data for assessing CRISPR activity from cells in bulk. In addition, methods of the invention offer a high throughput single cell analytical framework for generating single cell transcriptome data from which CRISPR activity may be evaluated.

## Description

### Technical Field

The invention relates to systems and methods for screening cells modified by gene-editing tools.

### Background

Genetic perturbation screens are a powerful tool for drug development. These screens employ a forward genetics approach, in which cellular phenotypes arising from genetic perturbations (e.g., a gene knockdown) are assessed. This approach can reveal causal relationships between genes and cellular phenotypes, which is useful for identifying key regulators of development and disease as potential drug targets.

Unfortunately, current methods have inherent limitations. For example, widely used methods of genetic perturbation screens are restricted to simple readouts of cell proliferation and cell sorting of fluorescently labeled molecules. Comprehensive molecular readouts, e.g., transcriptome profiling, are restricted to a much lower throughput because cells need to be physically separated for analysis. And although some methods exist for separating cells, i.e., microfluidics, those methods require expensive devices and are limited to processing cells individually, which inherently restricts the number of cells that can be analyzed in any one experiment.

### Summary

This disclosure provides pre-templated instant partitions useful for assessing transcriptional phenotypes of genetically perturbed cells on a large scale. The pre-templated instant partitions are formed in a fast, single-tube format that does not require expensive instrumentation or microfluidic consumables. Rather, the pre-templated instant partitions make use of hydrogel template particles that template the formation of a multitude (e.g., thousands or millions) of partitions simultaneously, in a single tube, and segregate single cells inside those partitions for single cell gene profiling. Each partition functions as an individual reaction chamber for profiling of single cells in parallel. Moreover, methods of the invention provide for the detection of each cell's guide RNA together with its transcriptome to identify transcriptional effects associated with genetic perturbations of cells. Accordingly, methods of the invention offer a massively parallel workflow for screening genetically perturbed cells on a large scale while eliminating drawbacks associated with existing single cell modalities. Such methods are useful for rapidly identifying genes or signaling pathways involved in disease and for assessing abilities of CRISPRs to produce desired effects before use in clinical treatments.

Methods of the invention are useful for performing genetic screens to systematically analyze gene function in cells, including patient cells. Methods of the invention provide for genetic screens that are performed in an array format on a large scale. In particular, methods of the invention provide for pooling of genetically perturbed cells, which are separately processed by their inclusion in different partitions. The readouts of these methods are useful for providing characterizing phenotypes of single cells, e.g., single cell expression of genes involved in growth or disease. These methods may be particularly applicable for interpreting complex gene pathways or the combined non-linear effects of multiple factors.

The invention provides systems and methods that are useful to quickly identify complex phenotypes, e.g., transcriptional profiles, of cells by combining CRISPRs with a high throughput, massively parallel approach of single cell gene profiling. In preferred embodiments, the CRISPRs are introduced into cells by plasmids encoding polyadenylated copies of guide RNA. The polyadenylated copies of guide RNA corresponds with guide RNA involved in genomic perturbations of the cells. Because the guide RNA copies are polyadenylated, they can be captured and identified with gene transcripts of the single cells. As such, methods of the invention offer comprehensive molecular readouts of genetically perturbed cells in bulk with a single cell transcriptome readout. These methods are useful to decrease the time and costs associated with assaying effects of large numbers of perturbations from cells individually.

Methods of the invention provide comprehensive assessments of genetically perturbed cells using methods that are high throughput and massively scalable. As such, methods of the invention are useful to analyze the effects of genetic perturbations across hundreds to thousands of genes in tens of thousands of cells from a single experiment. Unlike methods that use microfluidic devices, methods of the invention are easily scalable and capable of processing cells in parallel. Moreover, since methods of the invention do not involve expensive instrumentation or difficult sample preparation steps, methods of the invention can be rapidly integrated into existing analytical pipelines to quickly screen CRISPRs for desired gene perturbations, or interrogate complex gene networks involved in development or disease.

Methods of the invention enable pooled gene perturbation screens with single cell transcriptome readouts. Methods of the invention involve the insight that guide RNAs and their induced cellular response are compartmentalized inside cells. By using cells having detectable guide RNAs (i.e., polyadenylated guide RNAs), methods of the invention can compartmentalize single cells of pooled screens and generate single cell RNA sequencing libraries to identify guide RNAs and induced cellular responses of the cells. Preferably, the detectable guide RNAs are copies of the guide RNAs used to modify genomes of the cells, e.g., to cause a target deletion or insertion of nucleic acids. The detectable guide RNAs may be exact copies of the guide RNAs involved in gene modifications except for a polyadenylated sequence at a 3' end.

In one aspect, the invention provides a sample preparation method. The method involves compartmentalizing cells into individual compartments (i.e., partitions) using template particles. The template particles template the formation of partitions while simultaneously segregating single cells into distinct partitions for profiling pooled cells in parallel. Methods involve combining, in a vessel, template particles with cells in a first fluid, adding a second fluid that is immiscible with the first fluid to the first fluid, and agitating the fluids to generate the partitions. Preferably, the fluids are agitated by vortexing, which allows parameters of fluid agitation to be more easily controlled (e.g., by adjusting time or speed of the vortexer) and thus provides data that is more easily reproducible. Upon agitation, the partitions form inside the vessel nearly simultaneously. Advantageously, substantially all the partitions formed will include a single one of the template particles, and as such, in preferred embodiments the template particles are functionalized to introduce components into the partitions useful for preparing single cell RNA sequencing reactions, such as, for example, indexes or nucleic acid synthesis reagents.

After segregation into partitions, methods of the invention involve lysing the cells to release RNA from the cells for indexing. Indexing RNA includes providing the released RNA with cell-specific barcodes, thereby allowing RNA released from a particular cell to be associated together. In preferred embodiments, indexing involves hybridizing RNA with barcoded oligonucleotides introduced into the partitions by the template particles. Once RNA hybridizes with the oligonucleotides, the RNA is effectively indexed. The indexed RNA can then be processed to quantify RNA expression of single cells. In certain embodiments, RNA quantification data can be used to create gene expression profiles. The gene expression profiles can be used to identify characteristics of the CRISPR treated cells useful to, for example, assess CRISPR-driven gene edits and/or determine the effectiveness of a CRISPR design for eliciting a target knock down or knockout.

Methods of indexing preferably involve target capture of RNA molecules with capture sequences of oligonucleotides introduced into partitions by template particles. The capture of RNA molecules involves hybridization of the RNA molecules with complementary capture sequences. In certain embodiments, the oligonucleotides are linked to the template particles at a 5' end and the capture sequence is provided by, or near, a free 3' end of each oligonucleotide. The capture sequence may comprise a poly-T sequence to capture poly-A tails of the polyadenylated RNA, which is useful for whole genome profiling. Alternatively, the capture sequence may comprise a gene-specific complementary sequence (e.g., a sequence complementary to an oncogene). As such, methods of the invention can offer comprehensive insights of single cell whole transcriptome or targeted gene expression. The captured RNA can be reverse transcribed to create a cDNA library for sequencing. Preferably, the partitions are broken or ruptured before reverse transcribing the polyadenylated RNA to allow for one or more purification steps to reduce or eliminate background and thus improve data quality. The mRNA may be reverse transcribed into cDNA and simultaneously indexed, e.g., barcoded. The barcoded cDNA can be amplified to generate a plurality of barcoded amplicons. The amplicons can be sequenced by next generation sequencing methods, and because of the barcodes, each sequence read can be traced back to the cell. The sequence reads are processed to generate an expression profile for the cell.

After obtaining gene expression profiles from target cells, the profiles may be analyzed by comparing the profiles with reference or control profiles to ascertain information about the CRISPR-edited cells. In other instances, profiles of target cells can be compared to profiles derived from cells with certain phenotypes to determine whether the target cells share characteristics of the cells of the phenotype. This may be useful for determining whether a particular CRISPR design had a desired effect. In some instances, methods of the invention further include enriching, with a portion of the cDNA, for molecules of cDNA copied from the polyadenylated guide RNAs. Enriching may involve performing one or more rounds of PCR and may be useful for identification of guide RNAs associated with gene-edits of cells.

Methods of the invention provide a sample preparation process for direct detection of guide RNAs from single-cell transcriptome data. The method involves introducing, into cells, one or more CRISPR plasmids. The one or more CRISPR plasmids may include lentiviral plasmids and may be introduced into the cells via a lentivirus. The one or more plasmids preferably encode a Cas endonuclease, a guide RNA, and a polyadenylated guide RNA. In some embodiments, the Cas endonuclease, the guide RNA, and the polyadenylated guide RNA are all encoded by the same plasmid. The guide RNA is preferably encoded by a sequence of plasmid driven by an RNA polymerase III promoter, e.g., a human U6 promoter. Once integrated inside the cells, the guide RNA is transcribed by an RNA polymerase III enzyme. The polyadenylated guide RNA, however, is preferably transcribed in the cells by an RNA polymerase II enzyme. The RNA polymerase II enzyme provides the polyadenylated guide RNA with a polyadenylated sequence at a 3' end of the guide RNA. The polyadenylated guide RNA may have a sequence that is substantially identical to the guide RNA involved gene-editing and thus useful to easily identify guide RNA involved in editing of a cell.

In certain aspects, the invention provides a kit for single cell profiling of CRISPR-edited cells in bulk. The kit includes template particles comprising a plurality of oligonucleotides having capture sequences specific to one or more genes of interest. The kit may include various reagents for preparing and partitioning an emulsion. For example, the kit may provide reagents for stabilizing droplets formed inside an emulsion with the template particles. The kit may further include reagents for cell lysis and nucleic acid synthesis. A researcher following instructions provided by the kit can use template particles to screen cells treated with CRISPRs. The CRISPR screens made from kits of the invention may comprise single cell expression profiles of specific genes of interest, such as, oncogenes, and identities of guide RNAs associated with the cells. Template particles may be custom designed for the user's specific needs, for example, designed to include capture probe sequences specific to the certain genes of interest, such as oncogenes. The template particles may be shipped inside sample preparation tubes, or sample collection tubes, such as blood collection tubes. The template particles are preferably in a dried or lyophilized format.

### Brief Description of Drawings

FIG. 1 diagrams a library preparation method for screening cells.
FIG. 2 shows a template particle linked to oligos for capturing polyadenylated RNA.

### Detailed Description

This disclosure provides high-throughput systems and methods for screening genetically perturbed cells in bulk with single cell resolution. In particular, this disclosure provides pre-templated instant partitions useful for assessing phenotypes of genetically perturbed cells. The pre-templated instant partitions make use of hydrogel template particles for templating the formation of, for example, thousands to millions of partitions simultaneously, in a single tube, and segregate single cells inside those partitions for single cell gene profiling. Advantageously, each cell expresses guide RNA guide that is modified, e.g., polyadenylated, in the cell and therefore the RNA guide is also partitioned with the cell. As a result, both the RNA gene transcripts from the cells and a molecule identifying the guide RNA can be barcoded with a sequence unique to the partition. Accordingly, methods of the invention use cells expressing detectable versions of guide RNAs. Methods of the invention provide for the detection of each cell's guide RNA along with its single cell transcriptome, which is useful for assessing CRISPR activity from cells. Accordingly, methods of the invention provide comprehensive molecular readouts of genetically perturbed cells by copying guide RNAs into transcriptome data of cells.

The invention provides high-throughput systems and methods useful for screening cells perturbed by CRISPRs. The cells are preferably perturbed with CRISPRs plasmids that encode Cas endonuclease, guide RNA, and polyadenylated guide RNA. The Cas endonuclease functions with guide RNA to edit a genome of a cell, e.g., to create a deletion, an insertion, an inversion, etc., of nucleic acids present in a genome of a cell. As discussed in detail below, the polyadenylated guide RNA is designed to correspond to, e.g., report or identify, the guide RNA involved in gene editing. Since the guide RNA is polyadenylated, the guide RNAs can be captured with poly-T capture sequences of capture oligos and subsequently processed by standard RNA sequencing preparation methodologies. Detecting each cell's guide RNA along with its single cell transcriptome provides useful single cell expression data to assess genome modifications induced by CRISRPs. Accordingly, methods of the invention are useful to screen CRISPRs for desired knockdowns or knockouts or interrogate complex gene networks associated with certain diseases.

The invention makes use of hydrogel template particles that, when combined with in a vessel with immiscible fluids and agitated, template the formation of a multitude of droplets near simultaneously inside the vessel. During droplet formation, single ones of the CRISPR-edited cells are encapsulated into the individual droplets with single ones of the hydrogel particles. As such, the droplet-templating particles can also be used to introduce components into the droplets, such as barcoded oligonucleotides, for indexing or barcoding RNA molecules for single cell RNA sequencing. Each of the droplets functions as an individual reaction chamber for preparing a single cell RNA sequencing library. Methods of the invention can be used to prepare libraries for single cell analysis of, for example, at least 100 cells, at least 1000 cells, at least 1,000,000 cells, at least 2,000,000 cells, or more, from a single reaction tube. Accordingly, this approach provides a massively high throughput, analytical framework for screening CRISRPs with a workflow that is inexpensive, scalable, and accurate.

In some aspects, methods of the invention combine multiplexed CRISPR mediated gene editing with a high throughput single cell RNA-seq library preparation workflow to prepare libraries for assessing comprehensive gene expression phenotypes for each edited cell. In some embodiments, methods of the invention are useful to characterize a gene's function by applying genetic perturbations with CRISPRs to knock down or knock out one or more genes and studying the resulting phenotype. Methods of the invention are also useful to assess whether a particular CRISPR system, and in particular a guide RNA, is capable of eliciting a desired cellular response before use in a clinical application. Methods of the invention provide a reverse genetics approach that allows for the investigation of phenotypes at the level of the single cell transcriptomes, to elucidate gene functions in thousands to millions of cells, in a massively parallel format.

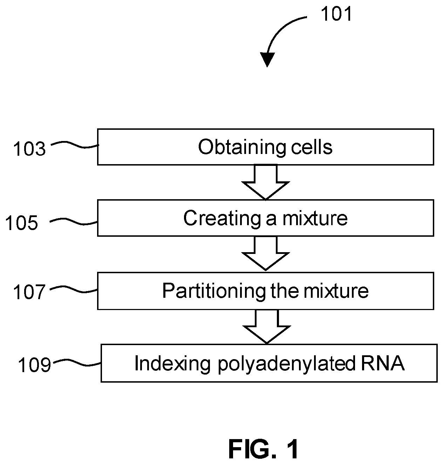

FIG. 1 diagrams a library preparation method 101 for screening cells. The method 101 includes obtaining cells 103 comprising polyadenylated guide RNA. As discussed below, the polyadenylated guide RNA contained in the cells is preferably indicative of guide RNA used to modify genomes of the cells. For example, the polyadenylated guide RNA may be an identical copy of guide RNA useful for directing a Cas endonuclease to a target sequence in the cell during gene-editing, as discussed below.

The method further includes creating a mixture 105 of the cells and template particles useful for partitioning the cells into distinct compartments. The mixture 105 may be created by adding template particles to a vessel containing the cells in an aqueous fluid (e.g., culture media, or saline), and then adding a second fluid (e.g., an oil), that is immiscible with the first fluid, to the vessel.

Next, the method then involves partitioning the mixture 107. Partitioning the mixture is preferably performed by vortexing the vessel. During vortexing, the mixture partitions 107 into aqueous droplets that each contain zero or one cell. In particular, vortexing produces a plurality of partitions (e.g., hundreds to thousands, etc.), wherein a substantial number of those partitions will include a single cell and a single template particle. For example, at least 25% of the partitions will include a single template particle and a single cell. Preferably, at least 80%, or 90% of the partitions will include a single template particle and a single cell.

The method then involves indexing 109 polyadenylated RNA released from the cells inside the droplets. Indexing 109 polyadenylated RNA may be accomplished by binding the released polyadenylated RNA with barcoded oligonucleotides provided by the template particles. For example, as described in more detail below, and also described in co-owned U.S. Pat. Appl. No. 17/146,720, incorporated by reference.

Methods of the invention provide useful tools for screening CRISPR-edited cells with a comprehensive molecular readout at a level of single cells.

CRISPR gene editing is a genetic engineering technique in molecular biology by which the genomes of living cells may be modified. Often CRISPRs involve the use of a Cas9 endonuclease, although other Cas endonucleases may be used (e.g., Cas12 or CasX) as the scope of the invention is not limited to a particular species of endonuclease. Preferably, Cas9 (or "CRISPR-associated protein 9") is used. Cas9 is an endonuclease enzyme that uses guide RNA sequences as a guide to recognize and cleave specific strands of DNA that are complementary to the guide RNA sequence. The guide RNA may be designed using methods known in the art to target Cas9 to a desired sequence of the genome. For example, as discussed in Hanna, 2020, Design and analysis of CRISPR-Cas experiments, Nature Biotechnology volume 38, pages 813-823, incorporated by reference. For example, from a list of genes or genetic sequences, scientists or clinicians may generate a list of CRISPR targets in a genome. There are preferably targets of ~20-base DNA sequences located in the genome adjacent to sites known as protospacer-adjacent motifs (PAMs). For CRISPR screening, it may be essential to knock out all the genes being studied. Therefore, to increase the probability of cutting, several target sites may be selected for every gene or genetic sequence being studied. For example, approximately 6-8 target sites per gene may be targeted. Each guide RNA used may include a corresponding polyadenylated guide RNA that is captured (i.e., with a poly-T capture sequence) by methods of the invention for RNA sequencing. The data generated by sequencing polyadenylated RNAs is useful to associate guide RNA used in editing of a cell with the resultant transcriptome induced by editing the cell.

Cas9 endonucleases, together with guide RNA, form the basis of a CRISPR system for editing cells of the invention. The system can be used to edit genes within cells or organisms. By delivering the Cas9 nuclease complexed with synthetic guide RNA into a cell (e.g., via a plasmid), the cell's genome can be cut at a desired location (complementary to the sequence of guide RNA), thereby allowing existing genes to be removed and/or new ones added in vivo. This technique allows for genomes of cells to be edited in vivo with extremely high precision, cheaply and with ease. It can be used in the creation of new medicines, therapeutic products, or as a means of controlling pathogens and pests.

Methods of the invention are useful for CRISPR screening. The basic idea of CRISPR screening is to knock out (e.g., delete or disrupt expression) genes that may be important. The intended result is a population of cells with a different gene knocked out in each cell in the dish. Some cells may die, but others will survive, or even grow better and become the predominant cell type. After the knockout the cells may be allowed to grow for a few days. After which, methods of the invention may be performed on the entire mixed population of pooled cells to determine which sequences are present and which are depleted or absent. Such experiments can be useful to identify genetic sequences necessary for survival under normal conditions. In other embodiments, the CRISPR screening studies may be useful for identification of genes that allow cells to survive under specific conditions, such as drug treatment or other physiological situations of interest.

The invention provides methods for screening CRIPSR-edited cells from single cell transcriptome data. In some aspects, methods of the invention rely on the direct detection of guide RNAs from the single cell transcriptome data. In certain embodiments, the guide RNAs are detectable from single cell transcriptome data by virtue of having a polyadenylated 3' tail. The presence of the polyadenylated 3' tail enables the guide RNA to be captured, e.g., by a poly-T region of an oligonucleotide, and processed for RNA sequencing.

Most CRISPR/Cas9 guide RNA expression systems use RNA polymerase III promoters, such as the U6 promoter. While efficient for generating guide RNAs for many genome editing applications, the guide RNAs transcribed by RNA polymerase III lack polyadenylated sequences. As such, expression of guide RNAs transcribed by RNA polymerase III are dissociated from cell transcriptome data.

To associate guide RNAs expressed by cells with the cell's transcriptome, methods of the invention employ plasmid in which guide RNAs are under the control of polymerase II promoters. Following transcription, the polymerase II guide RNAs are rapidly modified with a 3' poly-A tail and exported from the nucleus. Thus, modifications of the guide RNAs to incorporate the poly-A tail may alter localization of guide RNAs and thus impact performance. As such, methods of the invention preferably include plasmids encoding guide RNAs under the control of RNA polymerase III promoters, for effecting genome edits, and additionally, corresponding copy of guide RNAs under the control of a polymerase II promoters, for detection of the guide RNA by capture of the 3' poly-A tail. Preferably, the polyadenylated guide RNAs comprises sequences that are substantially identical to the guide RNAs used in gene-editing, for example, at least 75% identical.

The plasmids used in methods of the invention may comprise re-engineered plasmids of constructs demonstrated to be useful for pooled CRISPR screening, such as, the LentiGuide-Puro plasmid. The plasmid may be re-engineered to produce guide RNAs in polyadenylated mRNA transcripts. As such, the re-engineered plasmid preferably encode copies of guide RNAs under the control of polymerase II protomer. Because LentiGuide-Puro was originally derived from a lentiviral vector rendered self-inactivating by a 400-bp deletion of promoter elements within the 3' long terminal repeat, preferably, this position within the 3' long terminal repeat is used for the insertion of a guide RNA cassette. At this position, the guide RNA becomes part of the puromycin-resistance mRNA transcribed by RNA polymerase II and is detectable by RNA sequencing protocols using poly-A enrichment. Accordingly, methods of the invention thereby solve challenges of detecting guide RNAs in single-cell transcriptomes at the vector level, which makes it compatible with various single-cell RNA-seq assays and with widely used cloning protocols for pooled screening.

Plasmids may be used to express polyadenylated guide RNA in cells. For example, in preferred embodiments, the plasmids comprise a lentiviral construct including a guide RNA cassette within a 3' long terminal repeat, which may be duplicated during viral integration. Integration of the plasmid into cells causes the cells to express an RNA polymerase III transcript for genome editing, and a polyadenylated RNA polymerase II transcript detectable by single-cell RNA-sequencing. The plasmid may be introduced into cells by transfection methods. For example, the plasmid may be introduced into cells using a lentiviral transfection, for example, as described in Sano, 2020, Lentiviral CRISPR/Cas9-Mediated Genome Editing for the Study of Hematopoietic Cells in Disease Models J Vis Exp, 152, incorporated by reference.

In one aspect, this disclosure provides methods for preparing single cell RNA sequencing libraries from bulk cells. The cells may be obtained from a research or clinical facility. For example, the cells may be tissue culture cells, such as K562 cells. In other embodiments, the cells may include live cells obtained from, for example, a sample (tissue of bodily fluid) of a patient. The sample may include a fine needle aspirate, a biopsy, or a bodily fluid from the patient. Upon being isolated from the sample, the cells may be processed by, for example, generating a single cell suspension with an appropriate solution. Such solution will generally be a balanced salt solution, e.g. normal saline, PBS, Hank's balanced salt solution, etc.,

Preferably the cells express polyadenylated guide RNAs. The cells may be modified with plasmids to induce expression of CRISPRs (e.g., Cas9 and an associated guide RNA) and a polyadenylated version of the guide RNA. For example, the cells may be transfected with plasmids encoding guide RNAs under the control of a polymerase II promoter, for example, as described in Datlinger, 2017, Nat Methods, 14(3) 297-301, which is incorporated by reference.

The cells may be prepared for single cell analysis at a clinical facility and provided to a laboratory facility for performing steps of the method. As such, the cells may be obtained by post or mail in one or more cryopreservation vials on dry ice. The cryopreservation vials may include other materials, such as cellular debris released by ruptured cells. Advantageously, methods of the invention are useful for capturing material of interest, i.e., target RNAs, and separating the material of interest away from material that is not of interest, e.g., cell debris.

The method involves combining the cells with template particles useful for compartmentalizing the cells into separate partitions. The cells and template particles are combined in a first fluid. The first fluid may be an aqueous fluid, such as, media or a saline solution. The template particles and methods for leveraging particle-templated emulsification technology are described in, Hatori, 2018, Anal Chem, (90):9813-9820, which is incorporated by reference. Essentially, micron-scale beads, e.g., hydrogels, or template particles, are used to define an isolated fluid volume surrounded by an immiscible partitioning fluid. The partitions may be stabilized by the addition of one or more and temperature insensitive surfactants. The template particles of the present disclosure may be prepared using any method known in the art. Generally, the template particles are prepared by combining hydrogel material, e.g., agarose, alginate, a polyethylene glycol (PEG), a polyacrylamide (PAA), acrylate, acrylamide/bisacrylamide copolymer matrix, and combinations thereof. The template particles may comprise magnetic or paramagnetic particles embedded within the hydrogel for manipulating the hydrogels (e.g., moving) during library preparing. Following the formation of the template particles they are sized to the desired diameter. In some embodiments, sizing of the template particles is done by microfluidic co-flow into an immiscible oil phase.

In some embodiments, the cells are incubated with the template particles (e.g., for approximately 5 min at room temperature) to facilitate surface interactions between the template particles and the cells thereby improving capture of the cells into separate partitions upon shearing or vortexing the mixture.

After incubating, a second fluid that is immiscible with the first fluid is added to the mixture. The second fluid is preferably an oil. The second fluid may overlay the aqueous first fluid. In some embodiments, one or more surfactants, described below, may be added to the mixture to stabilize partitions.

The method then includes partitioning or shearing the fluids to generate monodisperse droplets, i.e., droplets. Preferably the mixture is partitioned by vortexing. Vortexing is preferred for its ability to reliably generate partitions of a uniform size distribution. Uniformity of partitions may be helpful to ensure each "reaction chamber" is provided with substantially equal reagents. Vortexing is also easily controlled (e.g., by controlling time and vortex speed) and thus produces data that are more easily reproducible. Vortexing may be performed with a standard bench-top vortexer or a vortexing device as described in co-owned U.S. Patent Application No. 17/146,768, which is incorporated by reference.

Alternatively, partitioning may comprise agitating the tube containing the fluids using any other method of controlled or uncontrolled agitation, such as shaking, pipetting, pumping, tapping, sonication and the like. After agitating (e.g., vortexing), a plurality (e.g., thousands, tens of thousands, hundreds of thousands, one million, two million, ten million, or more) of aqueous partitions is formed essentially simultaneously. Vortexing causes the fluids to partition into a plurality of monodisperse droplets. A substantial portion of droplets will contain a single template particle and a single target cell. Droplets containing more than one or none of a template particle or target cell can be removed, destroyed, or otherwise ignored

The next step of the method may be to lyse the pooled cells. Cell lysis may be induced by a stimulus, such as, for example, lytic reagents, detergents, or enzymes. Reagents to induce cell lysis may be provided by the template particles via internal compartments. In some embodiments, lysing involves heating the droplets to a temperature sufficient to release lytic reagents contained inside the template particles into the monodisperse droplets. This accomplishes cell lysis of the cells, thereby releasing mRNA inside of the droplets that contained the target cells for indexing.

RNA released from cells inside partitions is subsequently indexed or barcoded. Preferably, the released RNA includes polyadenylated RNA, including, polyadenylated guide RNA. Indexing is performed by hybridizing polyadenylated RNA with oligonucleotides of hydrogel template particles. The oligonucleotides are preferably attached to the template particles at a 5'end and include a capture sequence (e.g., a poly T sequence) at a free 3' end for target capture of polyadenylated RNA. The oligonucleotides may further include one or more barcodes for indexing the polyadenylated RNA. By hybridizing polyadenylated, molecules of polyadenylated RNA are effectively indexed. That is, the molecules of polyadenylated RNA become tagged with a molecular barcode, e.g., a random or not so random sequence of nucleic acids, that distinguishes polyadenylated RNA released from one cell from RNA released by a different cell.

After indexing, methods of the invention may include reverse transcribing captured polyadenylated RNA into double started cDNA, which is more stable than RNA. Reverse transcription preferably occurs outside of the partitions. As such, the partitions may be broken to release template particles comprising captured polyadenylated RNA for reverse transcription. To break the partitions, samples may be treated with a breaking buffer. Once broken, the template particles may be washed with a wash buffer (e.g., ethanol) and bound polyadenylated RNA may be reverse transcribed into cDNA containing one or more indexes or barcodes provided by template particles. The indexes or barcodes provide the cDNAs with cell identity information and unique molecular identifiers. The cDNA may also include primer sites for whole transcriptome amplification.

Accordingly, reverse transcription can be carried out to generate a library comprising cDNA with a barcode sequence that allows each library sequence to be traced back to the single cell from which the polyadenylated RNA was derived. In preferred embodiments, template particles isolated with the mRNA include a plurality of barcoded capture sequences that hybridize with target polyadenylated RNA. After hybridization, cDNA is synthesized by reverse transcription. Reagents for reverse transcription can be provided in a variety of ways in a variety of formats. In some instances, reagents and reverse transcriptase are provided by the template particles. Once a library is generated comprising barcoded cDNA, the cDNA can be amplified, by for example, PCR, to generate amplicons for sequencing.

Methods and systems of the invention use template particles to template the formation of monodisperse droplets and isolate single target cells. In preferred embodiments, the template particles of the invention are further useful to capture and index polyadenylated RNA, including polyadenylated guide RNA, for screening CRISPRs.

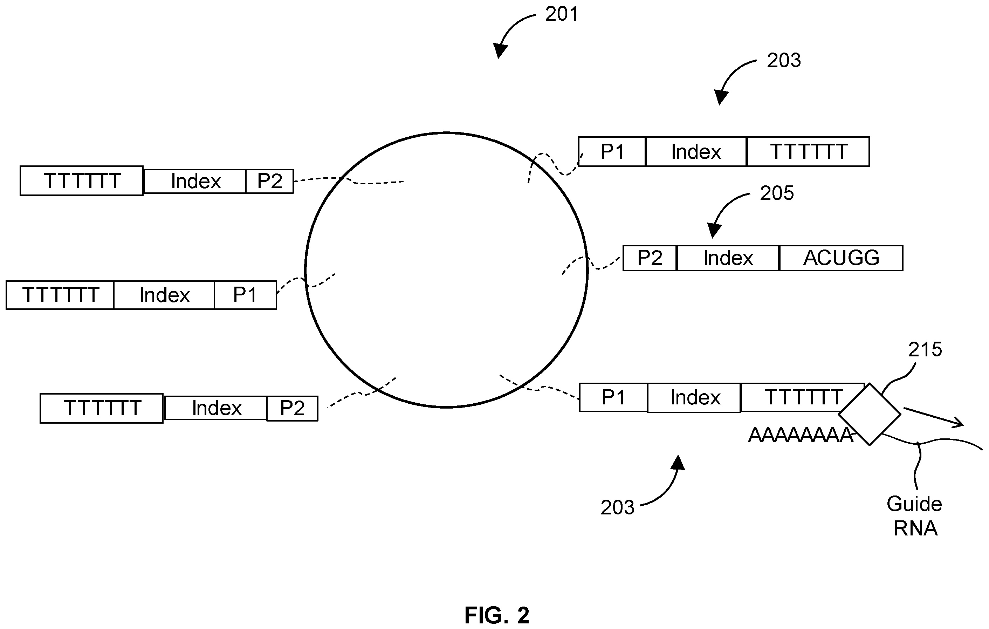

FIG. 2 shows a template particle 201 linked to oligonucleotides 203 for capturing polyadenylated RNA. The oligonucleotides are useful to initiate reverse transcription of the polyadenylated RNA. As shown, the template particle 201 is linked to (among other things) polyadenylated RNA capture probes, or oligonucleotides 203, that include a 3' poly-T capture region. Where the initial sample includes polyadenylated RNA, the capture region hybridizes by Watson-Crick base-pairing to poly-A tails of the polyadenylated RNA in the sample and thus provides as a primer for reverse transcriptase (illustrated by 215), to make a cDNA copy of the RNA.

A capture oligonucleotide hybridizes to polyadenylated RNA, including polyadenylated guide RNA, as illustrated. A reverse transcriptase 215 binds and initiates synthesis of a cDNA copy of the polyadenylated RNA. Note that the RNA is connected to the particle 201 non-covalently, by Watson-Crick base-pairing. The cDNA that is subsequently synthesized will be covalently linked to the particle 201 by virtue of phosphodiester bonds formed by reverse transcription.

In preferred embodiments, the captured RNAs are mRNAs. For example, methods of the disclosure may be used to make a cDNA library useful for showing an expression profile of a cell together with data useful to report on the identity of guide RNA involved in editing that cell. Where the target RNAs are mRNAs, the template particles may include mRNA capture oligos useful to at least synthesize a first cDNA copy of an mRNA. The particles may further include cDNA capture oligos with 3' portions that hybridize to cDNA copies of the mRNA. For the cDNA capture oligos, the 3' portions may include gene-specific sequences or hexamers.

Capture oligonucleotides of the invention may include, from 5' to 3', a binding site sequence P5, an index, and a poly-T segment. The cDNA capture oligos may include, from 5' to 3', a binding sequence P7 and a hexamer, or gene-specific sequence. Any suitable sequence may be used for the P5 and P7 binding sequences. For example, either or both of those may be arbitrary universal priming sequence (universal meaning that the sequence information is not specific to the naturally occurring genomic sequence being studied, but is instead suited to being amplified using a pair of cognate universal primers, by design).

Indexes of capture oligonucleotides may be any suitable barcode or index such as may be useful in downstream information processing. It is contemplated that the P5 sequences, the P7 sequence, and the index segment may be the sequences use in NGS indexed sequences such as performed on an NGS instrument sold under the trademark ILLUMINA, and as described in Bowman, 2013, Multiplexed Illumina sequencing libraries from picogram quantities of DNA, BMC Genomics 14:46, incorporated by reference. The hexamer segments may be random hexamers or selective hexamers (aka not-so-random hexamers).

Some embodiments of the invention make use of not-so-random (NSR) oligomers (NSROs). See Armour, 2009, Digital transcriptome profiling using selective hexamer priming for cDNA synthesis, Nat Meth 6(9):647-650, incorporated by reference. Preferably, the particles are linked to capture oligos that include one or more primer binding sequences P5, P7 cognate to PCR primers that may be used in an option downstream amplifying step (such as PCR or bridge amplification).

The template particles may provide oligonucleotides for target capture and barcoding of polyadenylated RNA. Barcodes specific to each template particle may be any group of nucleotides or oligonucleotide sequences that are distinguishable from other barcodes within the group. Accordingly, a PIP encapsulating a template particle and a single cell provides to each nucleic acid molecule released from the single cell the same barcode from the group of barcodes. The barcodes provided by template particles are unique to that template particle and distinguishable from the barcodes provided to nucleic acid molecules by every other template particle. Once sequenced, by using the barcode sequence, the nucleic acid molecules can be traced back to the single cell based on the barcode provided by the template particle that the single cell was partitioned with. Barcodes may be of any suitable length sufficient to distinguish the barcode from other barcodes. For example, a barcode may have a length of 4, 5, 6, 7, 8, 9, 10, 11, 12, 13, 14, 15, 16, 17, 18, 19, 20, 21, 22, 23, 24, 25 nucleotides, or more.

The barcodes unique to each template particle may be pre-defined, degenerate, and/or selected at random. Barcodes may be added to nucleic acid molecules by "tagging" the nucleic acid molecules with the barcode. Tagging may be performed using any known method for barcode addition, for example direct ligation of barcodes to one or more of the ends of each nucleic acid molecule. Nucleic acid molecules may, for example, be end repaired in order to allow for direct or blunt-ended ligation of the barcodes. Barcodes may also be added to nucleic acid molecules through first or second strand synthesis, for example using capture probes, as described herein below.

In some methods of the invention, an index or barcode sequence may comprise unique molecule identifiers (UMIs). UMIs are a type of barcode that may be provided to a sample to make each nucleic acid molecule, together with its barcode, unique, or nearly unique. This may be accomplished by adding one or more UMIs to one or more capture probes of the present invention. By selecting an appropriate number of UMIs, every nucleic acid molecule in the sample, together with its UMI, will be unique or nearly unique.

UMIs are advantageous in that they can be used to correct for errors created during amplification, such as amplification bias or incorrect base pairing during amplification. For example, when using UMIs, because every nucleic acid molecule in a sample together with its UMI or UMIs is unique or nearly unique, after amplification and sequencing, molecules with identical sequences may be considered to refer to the same starting nucleic acid molecule, thereby reducing amplification bias. Methods for error correction using UMIs are described in Karlsson et al., 2016, Counting Molecules in cell-free DNA and single cells RNA", Karolinska Institutet, Stockholm Sweden, incorporated herein by reference.

In some embodiments of the template particles, a variation in diameter or largest dimension of the template particles such that at least 50% or more, e.g., 60% or more, 70% or more, 80% or more, 90% or more, 95% or more, or 99% or more of the template particles vary in diameter or largest dimension by less than a factor of 10, e.g., less than a factor of 5, less than a factor of 4, less than a factor of 3, less than a factor of 2, less than a factor of 1.5, less than a factor of 1.4, less than a factor of 1.3, less than a factor of 1.2, less than a factor of 1.1, less than a factor of 1.05, or less than a factor of 1.01.

Template particles may be porous or nonporous. In any suitable embodiment herein, template particles may include microcompartments (also referred to herein as "internal compartment"), which may contain additional components and/or reagents, e.g., additional components and/or reagents that may be releasable into monodisperse droplets as described herein. Template particles may include a polymer, e.g., a hydrogel. Template particles generally range from about 0.1 to about 1000 µm in diameter or larger dimension. In some embodiments, template particles have a diameter or largest dimension of about 1.0 µm to 1000 µm, inclusive, such as 1.0 µm to 750 µm, 1.0 µm to 500 µm, 1.0 µm to 250 µm, 1.0 µm to 200 µm, 1.0 µm to 150 µm 1.0 µm to 100 µm, 1.0µm to 10 µm, or 1.0 µm to 5 µm, inclusive. In some embodiments, template particles have a diameter or largest dimension of about 10 µm to about 200 µm, e.g., about 10 µm to about 150 µm, about 10 µm to about 125 µm, or about 10 µm to about 100 µm.

In practicing the methods as described herein, the composition and nature of the template particles may vary. For instance, in certain aspects, the template particles may be microgel particles that are micron-scale spheres of gel matrix. In some embodiments, the microgels are composed of a hydrophilic polymer that is soluble in water, including alginate or agarose. In other embodiments, the microgels are composed of a lipophilic microgel. In other aspects, the template particles may be a hydrogel. In certain embodiments, the hydrogel is selected from naturally derived materials, synthetically derived materials and combinations thereof. Examples of hydrogels include, but are not limited to, collagen, hyaluronan, chitosan, fibrin, gelatin, alginate, agarose, chondroitin sulfate, polyacrylamide, polyethylene glycol (PEG), polyvinyl alcohol (PVA), acrylamide/bisacrylamide copolymer matrix, polyacrylamide /poly(acrylic acid) (PAA), hydroxyethyl methacrylate (HEMA), poly N- isopropylacrylamide (NIPAM), and polyanhydrides, poly(propylene fumarate) (PPF).

In some embodiments, the presently disclosed template particles further comprise materials which provide the template particles with a positive surface charge, or an increased positive surface charge. Such materials may be without limitation poly-lysine or Polyethyleneimine, or combinations thereof. This may increase the chances of association between the template particle and, for example, a cell which generally have a mostly negatively charged membrane.

Other strategies may be used to increase the chances of templet particle-target cell association, which include creation of specific template particle geometry. For example, in some embodiments, the template particles may have a general spherical shape but the shape may contain features such as flat surfaces, craters, grooves, protrusions, and other irregularities in the spherical shape.

Any one of the above described strategies and methods, or combinations thereof may be used in the practice of the presently disclosed template particles and method for targeted library preparation thereof. Methods for generation of template particles, and template particles-based encapsulations, were described in International Patent Publication WO 2019/139650, which is incorporated herein by reference.

Methods of the invention generally relate to analysis and sequencing of gene transcripts from single cells modified by RNA guides in genomic areas of interest, for example oncogenes. Thus, PCR amplification of products derived from nucleic acid molecules released by single cells can be used to determine a gene expression profile for a cell for preselected gene mutations, e.g., mutations associated with cancer. For example, identification of a gene or mutation of interest may provide information that the cell from which the nucleic acid molecule was released is expressing gene transcripts associated with cancer as a result of the genomic modification resulting from the RNA guide. Because each nucleic acid molecule is tagged with a barcode unique to the PIP and single cell from which it was released, any gene transcript can be traced back to the PIP and single cell, thereby allowing for the identification of a RNA guide and genotypic modification created by the RNA guide.

For RNA or mRNA sequencing, sequencing may first comprise the step of preparing a cDNA library from barcoded RNA, through reverse transcription, and sequencing the cDNA. RNA sequencing may advantageously allow for the quantification of gene expression within the single cell, and can be used to identify characteristics of the single cell that can be used to, for example, make a diagnosis, prognosis, or determine drug effectiveness. Reverse transcription of cDNA molecules from RNA can be performed both within the PIP or after barcoded RNA molecules have been released from each PIP.

Reverse transcription may be performed using without limitation dNTPs (mix of the nucleotides dATP, dCTP, dGTP and dTTP), buffer/s, detergent/s, or solvent/s, as required, and suitable enzyme such as polymerase or reverse transcriptase. The polymerase used may be a DNA polymerase, and may be selected from Taq DNA polymerase, Phusion polymerase (as provided by Thermo Fisher Scientific, Waltham, Massachusetts), or Q5 polymerase. Nucleic acid amplification reagents are commercially available, and may be purchased from, for example, New England Biolabs, Ipswich, MA, USA. The reverse transcriptase used in the presently disclosed targeted library preparation method may be for example, maxima reverse transcriptase. In some embodiments, the general parameters of the reverse transcription reaction comprise an incubation of about 15 minutes at 25 degrees and a subsequent incubation of about 90 minutes at 52 degrees.

Reverse transcription, for example where polyadenylated RNA (including polyadenylated guide RNA) is reverse transcribed, may comprise use of a capture sequence, e.g., poly-T sequence, and a capture primer or probe. Generally, a capture probe is an oligonucleotide. The capture probes may attach to the template particle's material via covalent acrylic linkages. The capture probes may comprise an acrydite-modified on their 5' end (linker region). Generally, acrydite-modified oligonucleotides can be incorporated, stoichiometrically, into hydrogels such as polyacrylamide, using standard free radical polymerization chemistry, where the double bond in the acrydite group reacts with other activated double bond containing compounds such as acrylamide. Specifically, copolymerization of the acrydite-modified capture probes with acrylamide including a crosslinker, e.g. N,N'-Methylenebis, will result in a crosslinked gel material comprising covalently attached capture probes. Capture probes may also comprise Acrylate terminated hydrocarbon linker and combining the said capture probes with a template particle will cause their attachment to the template particle.

After reverse transcribing RNA into cDNA, methods of the invention may involve amplifying the cDNA and then sequencing the amplicons for the analysis of genetic perturbations of cells.

Sequencing nucleic acid molecules may be performed by methods known in the art. For example, see, generally, Quail, et al., 2012, A tale of three next generation sequencing platforms: comparison of Ion Torrent, Pacific Biosciences and Illumina MiSeq sequencers, BMC Genomics 13:341. Nucleic acid molecule sequencing techniques include classic dideoxy sequencing reactions (Sanger method) using labeled terminators or primers and gel separation in slab or capillary, or preferably, next generation sequencing methods. For example, sequencing may be performed according to technologies described in U.S. Pub. 2011/0009278, U.S. Pub. 2007/0114362, U.S. Pub. 2006/0024681, U.S. Pub. 2006/0292611, U.S. Pat. 7,960,120, U.S. Pat. 7,835,871, U.S. Pat. 7,232,656, U.S. Pat. 7,598,035, U.S. Pat. 6,306,597, U.S. Pat. 6,210,891, U.S. Pat. 6,828,100, U.S. Pat. 6,833,246, and U.S. Pat. 6,911,345, each incorporated by reference.

One pipeline for processing sequencing data includes generating FASTQ-format files that contain reads sequenced from a next generation sequencing platform, aligning these reads to an annotated reference genome, and quantifying expression of genes. These steps are routinely performed using known computer algorithms, which a person skilled in the art will recognize can be used for executing steps of the present invention. For example, see Kukurba, Cold Spring Harb Protoc, 2015 (11):951-969, incorporated by reference.

Nucleic Acid molecules may advantageously be amplified prior to sequencing. Amplification may comprise methods for creating copies of nucleic acids by using thermal cycling to expose reactants to repeated cycles of heating and cooling, and to permit different temperature-dependent reactions (e.g. by Polymerase chain reaction (PCR). Any suitable PCR method known in the art may be used in connection with the presently described methods. Non limiting examples of PCR reactions include real-time PCR, nested PCR, multiplex PCR, quantitative PCR or touchdown PCR. Notably, each amplified copy of the nucleic acid molecule will comprise the barcode unique to a droplet for identifying the droplet and cell form which the nucleic acid molecule was released. Methods for amplification include whole genome amplification.

In some instances, methods of the invention may be useful for creating gene expression profiles identifying gene perturbations caused by CRISPR activity. Gene expression profiling is the measurement of the activity of the activity of genes to create a global picture of cellular function. Gene expression profiling includes the identification and measurement of quantity of mRNAs in the cell to measure the activity of the corresponding genes. While sequencing a genome provides information as to what the cell could possibly do, the expression profile provides information as to what the cell is actually doing at a point in time.

At any moment each cell makes mRNA from only a fraction of the genes it carries. If a gene is used to produce mRNA, it is considered "on", otherwise it is considered "off". Gene expression profiling may include measuring the relative amount of mRNA expressed in two or more conditions. For example, cells may be modified by an RNA guide that is thought to produce an "on" switch in a gene, an RNA guide that is thought to produce an "off" switch in a gene, and an RNA guide that is thought to produce no change in the gene. The gene expression profile provides information as to what the changes made by the guide RNAs in DNA actually result in phenotypically in the cell. Gene expression profiling may also provide information as the editing capacity of RNA guides, for example when multiple RNA guides targeting the same "on" switch are analyzed in parallel to assess varying levels of gene expression level changes.

Gene expression profiling is useful for analyzing genetic diseases with varying disease states, for example cancers, neurodegenerative diseases, neuropsychiatric disease, metabolic disorders, and cardiovascular disorders. Metabolic disorders may include type 2 diabetes and obesity. Cardiovascular disorders may include atherosclerosis and hypertension. Neurological disorders may include Alzheimer's or Parkinson's. Cancers may include Hodgkin lymphoma, non-Hodgkin lymphoma, myelodysplastic syndromes, breast cancer, prostate cancer, melanoma, ovarian cancer, sarcoma, oral carcinoma, or a hepatocellular carcinoma. Moreover, gene expression profiling can be useful for identifying the mechanisms of action of therapeutic interventions, for example small molecule drugs.

Methods of the invention combine CRIPSRs with high throughput single cell analysis. CRISPR systems involve RNA guided endonucleases. An endonuclease is an enzyme that cleaves the phosphodiester bond within a polynucleotide gene, for example genomic DNA. Endonucleases can cleave DNA relatively non-specifically or at very specific nucleotide sequences. RNA guided endonucleases are endonucleases that use an RNA guide to target a very specific location on DNA. In this way, the RNA guide or guide RNA, used herein interchangeably, confers target sequence specificity to the RNA guided endonuclease.

Cas9 is an RNA guided endonuclease that plays a vital role in the immunological defense of certain bacteria against DNA viruses. CRISPR-Cas9 is a gene editing system that is more specifically a dual-RNA guided DNA endonuclease. Cas9 interrogates DNA by checking for sites complementary to a 20 base pair spacer region on an RNA guide. If the DNA substrate is complementary to the guide RNA, Cas9 cleaves the DNA. Advantageously, Cas9 can cleave nearly any sequence complementary to the guide RNA. Cas9 is considered a dual-RNA guided DNA endonuclease because native Cas9 requires a guide RNA composed of two RNAs that are associated, a CRISPR RNA (crRNA) and a trans-activating crRNA (tracrRNA). Cas9 targeting is frequently simplified by using a single guide RNA (sgRNA) that combines the crRNA and tracrRNA into a single RNA molecule. RNA guides include crRNAs, tracrRNAs, guide RNA spacers, and sgRNAs.

Typically, CRISPR-Cas9 targeting specificity is determined by the 20 base pair sequence at the 5' end of the RNA guide. The desired target sequence must precede a protospacer adjacent motif, which is a short DNA sequence typically 2-6 Base pair in length that is typically 3-4 nucleotides downstream of the DNA region targeted for modification. After base pairing of the RNA guide to the target, Cas9 mediates a double strand break at about 3 base pair upstream of the protospacer adjacent motif.

RNA guided endonucleases may introduce gene knock out or knock in depending the double strand repair pathway. RNA guided endonucleases may also upregulate expression of a gene or gene transcripts by knocking out a repressor gene or downregulate expression of a gene or gene transcript by knocking out a promoter of the gene.

### Incorporation by Reference

References and citations to other documents, such as patents, patent applications, patent publications, journals, books, papers, web contents, have been made throughout this disclosure. All such documents are hereby incorporated herein by reference in their entirety for all purposes.

### Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention in its various embodiments and equivalents thereof.
Embodiments of the invention may include the features of the following enumerated paragraphs ("paras")
1. A library preparation method for screening single cells, the method comprising:
   obtaining cells comprising polyadenylated guide RNAs;
   combining, into a mixture, the cells with template particles comprising oligonucleotides;
   agitating the mixture to generate a plurality of partitions, near simultaneously, wherein a substantial portion of the partitions contain a single cell and a single template particle;
   releasing polyadenylated RNAs inside the partitions from the cells, wherein a portion of the polyadenylated RNAs comprises the polyadenylated guide RNAs; and
   indexing the polyadenylated RNAs inside the partitions with the oligonucleotides to produce a library of indexed nucleic acids for screening single cells.
2. The method of para 1, further comprising reverse transcribing the polyadenylated RNAs to create a cDNA library for sequencing.
3. The method of para 2, wherein reverse transcribing is performed after breaking the partitions.
4. The method of para 1, wherein the polyadenylated guide RNAs are encoded by one or more plasmids introduced into the cells by transfection.
5. The method of para 4, wherein the one or more plasmids further encode Cas endonucleases and guide RNAs for editing genomes of the cells.
6. The method of para 5, wherein the guide RNAs are transcribed in the cells by RNA polymerase III.
7. The method of para 5, wherein the polyadenylated guide RNAs are transcribed in the cells by RNA polymerase II.
8. The method of para 5, wherein the guide RNAs and the polyadenylated guide RNAs are encoded by the same plasmids.
9. The method of para 1, wherein the polyadenylated guide RNAs comprise a sequence that is substantially identical to the guide RNAs of each cell.
10. The method of para 1, wherein partitioning the mixture comprises vortexing.
11. The method of para 1, wherein the oligonucleotides comprise one or more primers for performing whole transcriptome amplification.
12. The method of para 2, further comprising enriching, with a portion of the cDNA, for molecules of cDNA copied from the polyadenylated guide RNAs.
13. The method of para 12, wherein enriching comprises performing one or more rounds of PCR.
14. The method of para 5, wherein the guide RNA is encoded by a sequence of the one or more plasmids driven by an RNA polymerase III promoter.
15. The method of para 14, wherein the promoter is a human U6 promoter.
16. The method of para 1, wherein the oligonucleotides comprise one or more barcodes.
17. The method of para 16, wherein at least one of the barcodes comprises a unique molecular identifier.
18. The method of para 1, wherein the oligonucleotides are attached to the template particles.
19. The method of para 5, wherein the guide RNAs comprise targeting sequences complementary to one or more genes.
20. The method of para 19, wherein the one or more genes include an oncogene.

## Claims

1. A library preparation method for screening single cells, the method comprising:
obtaining cells comprising polyadenylated guide RNAs, wherein the polyadenylated guide RNAs are encoded by one or more plasmids introduced into the cells by transfection and wherein the one or more plasmids further encode Cas endonucleases and guide RNAs for editing genomes of the cells;
combining, into a mixture, the cells with hydrogel template particles comprising oligonucleotides and magnetic particles, wherein the magnetic particles are embedded within the hydrogel, and wherein the oligonucleotides comprise one or more barcodes;
agitating the mixture to generate a plurality of partitions, simultaneously, wherein a portion of the partitions contain a single cell and a single hydrogel template particle;
releasing polyadenylated RNAs inside the partitions from the cells, wherein a portion of the polyadenylated RNAs comprises the polyadenylated guide RNAs;
indexing the polyadenylated RNAs inside the partitions with the oligonucleotides to produce a library of indexed nucleic acids for screening single cells;
breaking the partitions;
reverse transcribing the polyadenylated RNAs to create a cDNA library for sequencing, wherein the reverse transcribing is performed after breaking the partitions; and
manipulating the hydrogel template particles via the magnetic particles during the library preparation method.

2. The method of claim 1, wherein library preparation method does not comprise the use of a microfluidic device.

3. The method of claim 1, wherein the guide RNAs are transcribed in the cells by RNA polymerase III, wherein the polyadenylated guide RNAs are transcribed in the cells by RNA polymerase II, or wherein the guide RNAs and the polyadenylated guide RNAs are encoded by the same plasmids.

4. The method of claim 1, wherein the polyadenylated guide RNAs comprise a sequence that is substantially identical to the guide RNAs of each cell.

5. The method of claim 1, wherein partitioning the mixture comprises vortexing.

6. The method of claim 1, wherein the oligonucleotides comprise one or more primers for performing whole transcriptome amplification.

7. The method of claim 1, further comprising enriching, with a portion of the cDNA, for molecules of cDNA copied from the polyadenylated guide RNAs, optionally wherein enriching comprises performing one or more rounds of PCR.

8. The method of claim 1, wherein the oligonucleotides are attached to the template particles.

9. The method of claim 1, wherein the guide RNAs comprise targeting sequences complementary to one or more genes, optionally wherein the one or more genes include an oncogene.

10. The method of claim 1, wherein releasing comprises heating the partitions to a temperature sufficient to release lytic reagents contained inside the hydrogel template particles from the hydrogel template particles and into the partitions.

11. The method of claim 10, wherein the lytic reagents include detergents or enzymes are provided within internal compartments of the template particles.

12. The method of claim 1, wherein the hydrogel template particles comprise materials that provide the template particles with a positive surface charge to promote associating of cells with the hydrogel template particles.

13. The method of claim 12, wherein the materials include poly-lysine, polyethyleneimine, or a combination thereof.

14. The method of claim 1, wherein the magnetic particles are paramagnetic particles.

15. The method of claim 1, wherein the barcodes comprises unique molecular identifiers.
